# EUROPEAN PATENT APPLICATION

(11) **EP 4 600 491 A1**
(43) Date of publication of application: **13.08.2025**
(21) Application number: 23874070.8
(22) Date of filing: 27.06.2023
(51) Int. Cl.: F04B 37/10, F04B 35/04, F04B 39/00, A61M 1/06

(54) **BREAST PUMP DRIVING APPARATUS**

(30) Priority: 08.10.2022 CN 202211230322
(71) Applicant: Guangdong Youju Advanced New Materials Co., Ltd., Jiangmen, Guangdong 529040 (CN); Guangdong Golden Baby Health Supplies Co., Ltd., Jiangmen, Guangdong 529040 (CN)
(72) Inventor: NI, Jinxiang, Jiangmen, Guangdong 529040 (CN); WANG, Xianwen, Jiangmen, Guangdong 529040 (CN); LI, Haoyuan, Jiangmen, Guangdong 529040 (CN)
(74) Representative: Elzaburu S.L.P.
(86) International application number: PCT/CN2023/102833
(87) International publication number: WO 2024/074059

(57) **Abstract**

A breast pump driving apparatus is provided. A stepper motor (3)is provided at a housing having an open end through a bracket (2); an output shaft of the stepper motor (3)is a screw (5) and connected to a piston through a screw nut (7); the other end of the housing (1) is provided with an air tube joint (11) and a one-way valve to cooperate with an inner cavity of the housing (1) to generate different air pressures; and the bracket is provided with a muffling airway for reducing working noise. The breast pump driving apparatus changes the air pressure by driving the piston to move through the stepper motor (3) and the screw-nut (7) pair, achieving precise action and stable suction, and basically avoiding flow attenuation, thereby ensuring accurate suction and improving user comfort. Meanwhile, the breast pump driving apparatus has good working stability and low requirements for airtightness, reduces the failure rate, and improves the convenience of use. Furthermore, the breast pump ensures extremely low working noise, reducing the impact of working noise on the nursing mother and improving user comfort.

## Description

### TECHNICAL FIELD

The present invention relates to a breast pump, and in particular to a driving apparatus in a breast pump.

### BACKGROUNDTECHNOLOGY

Breast pumps are designed to extract milk from breasts, and are categorized into manual and electric types. As a critical component of the electric breast pump, an air pump is configured to control air pressure. Prior air pumps typically include direct current motor (DCM)-driven diaphragm air pumps and gear-reduction DCM-driven piston pumps. In the DCM-driven diaphragm air pump, the DCM rotates to squeeze and pull the diaphragm, thereby driving air flow and creating a negative pressure in the enclosed space connected to the air inlet. In the gear-reduction DCM-driven piston pump, the DCM rotates forward and backward to drive the screw to move, thereby achieving reciprocating motion of the cylinder. The DCM-driven diaphragm air pump has a small air flow rate, and there is significant flow attenuation during the increase of the negative pressure, making it hard for the air pumpto meet the operational need for large and precise suction. In addition, due to poor working stability, the DCM-driven diaphragm air pump cannot guarantee stable suction and working accuracy,thereby leading to poor user experience. The gear-reduction DCM-driven piston pump has a complex structure and a high failure rate, and its operation requires extremely high air tightness for various parts of the breast pump. When the breast pump is in use, a slight deviation in the fit between the flange of the milk bottle and the breast can cause program malfunctions, resulting in the inability of the breast pump to function. In addition, in prior breast pumps, the motor of the air pump is connected to the piston of the cylinder through the gear set and the speedchanger, and the rotation angle of the motor is detected by the grating, resulting in low working accuracy and high noise. Moreover, the structure can easily cause an imbalance of negative pressure inside the air pump, thereby affecting the motor speed. Furthermore, the high-speed DCM has a short carbon brush life and is prone to sparking faults.

### CONTENT OF THE INVENTION

An objective of the present invention is to provide a breast pump driving apparatus with a simple structure and accurate and stable operation, in order to improve the accuracy and comfort of breast pumping operations.

In the present invention, the breast pump driving apparatus includes a housing that is cylindrical and has an open end, where a motor is provided at the open end of the housing through a bracket; the housing is provided with a hood for covering the stepper motor; the motor is a stepper motor; an output shaft of the stepper motor is a screw; a piston moving along an inner wall of the housing is threaded to the screw through a screw nut; an end of the housing facing away from the stepper motor is provided with an air tube joint; and the air tube joint is provided with a throughh hole communicated with an inner cavity of the housing.

Furthermore, the end of the housing facing away from the stepper motor is provided with an exhaust hole; the exhaust hole is provided with a one-way valve for allowing only air to be discharged outward; a limit switch is provided in the housing; the stepper motor is started to drive the piston to move to a set position; and when the limit switch detects that the piston is in place, a control system starts working.

Furthermore, the one-way valve includes a flexible diaphragm and a pressure plate for fixing the diaphragm to the housing; the diaphragm is annular, and a sealing part is provided at a middle of the diaphragm and configured to seal the exhaust hole of the housing; the pressure plate is pressed against an annular edge of the diaphragm; the diaphragm is provided with a diaphragm hole located between the edge and the sealing part; and the pressure plate is provided with a pressure plate hole for communicating two sides inside.

Furthermore, the piston includes a piston rod and a piston head; an end of the piston rod is provided with a piston hole for the screw to extend into; and the screw nut is fixedly provided at the piston hole.

Furthermore, a side wall of the piston head is provided with an annular positioning groove recessed inwardly; a sealing ring is provided in the positioning groove; and the sealing ring hermetically abuts againstthe inner wall of the housing.

Furthermore, the bracket is sleeved on the piston rod; a positioning slot and a positioning rib cooperating with each other are provided between an inner side of the bracket and an outer wall of the piston rod; and the positioning slot and the positioning rib are parallel to an axial direction of the housing.

Furthermore, an end of the bracket is hermetically connected to the open end of the housing; a muffling airway is provided in the end of the bracket and configured to reduce a volume of noise; and sides of the end of the bracket facing and away from the piston are respectively provided with an airway inlet and an airway outlet that are communicated with the muffling airway.

Furthermore, the muffling airway includes a plurality of muffling chambers that are sequentially connected, with a tail-end muffling chamber communicated with the airway outlet; walls of all the muffling chambers facing the airway inlet are respectively provided with muffling holes; and all the muffling holes face respective outer sides of the muffling chambers.

Furthermore, the muffling chamber includes "<"-shaped walls facing and away from the airway inlet; upper and lower ends of the "<"-shaped wall are respectively connected to inner and outer walls of the muffling chamber; the muffling hole is located in an upper part of the "<"-shaped wall; a midpoint position of the "<"-shaped wall of the muffling chamber facing away from the airway inlet is provided with a muffling plate extending towards the inner wall of the muffling chamber; a gap is formed between the muffling plate and the inner wall of the muffling chamber; and a width of the gap is not greater than one tenth of a width of the muffling chamber.

Furthermore, the airway outlet is located at a bottom of the tail-end muffling chamber adjacent to an outer wall of the tail-end muffling chamber; the lower end of the "<"-shaped wall of the tail-end muffling chamber facing the airway inlet is provided with a partition extending towards another wall.

In the breast pump driving apparatus of the present invention, the housing is hollow inside. The piston is hermetically connected to the inner wall of the housing through a sealing ring, and moves along the inner wall of the housing under the drive of the motor, thereby changing the volume of an inner cavity of the housing and achieving suction and exhaust. The motor is a stepper motor, which is connected to the piston through a screw-nut pair, allowing the piston to precisely control its stroke and change the volume of the inner cavity to form a specific suction. The air tube joint on the housing is connected to a milk bottle through an air tube, and a flange of the milk bottle fits tightly with a breast to form a seal. When the stepper motor drives the piston to move towards one end of the stepper motor, a negative pressure is generated inside the inner cavity of the housing, the air tube, and the milk bottle that are communicated with each other, thereby forming a suction to suck up breast milk. When the stepper motor drives the piston to move away from the stepper motor, the negative pressure inside the housing is reduced until it is equal to the atmospheric pressure, restoring air pressure balance between the housing and the milk bottle to wait for a next milk suction action. The main sources that generate noise in the breast pump driving apparatus are the screw-nut pair, the piston, and the airflow inside the housing. Considering this, the end of the bracket is sealed with the housing to encapsulate the main sources that generate noise inside theinner cavity, effectively reducing the working noise of the breast pump. Meanwhile, through the muffling airway on the bracket, the sound wave and airflow undergo a noise reduction procedure through the muffling airway and are discharged out of the housing through the airway outlet, thereby further reducing the volume of the noise. The present invention has the following beneficial effects. The breast pump driving apparatus changes the air pressure by driving the piston to move through the stepper motor and the screw-nut pair, achieving precise action and stable suction, and basically avoiding flow attenuation, thereby ensuring accurate suction and improving user comfort. Meanwhile, the breast pump driving apparatus has good working stability and low requirements for airtightness, reduces the failure rate, and improves the convenience of use. Furthermore, the breast pump ensures extremely low working noise, reducing the impact of working noise on the nursing mother and improving user comfort.

### DESCRIPTION OF THE DRAWINGS

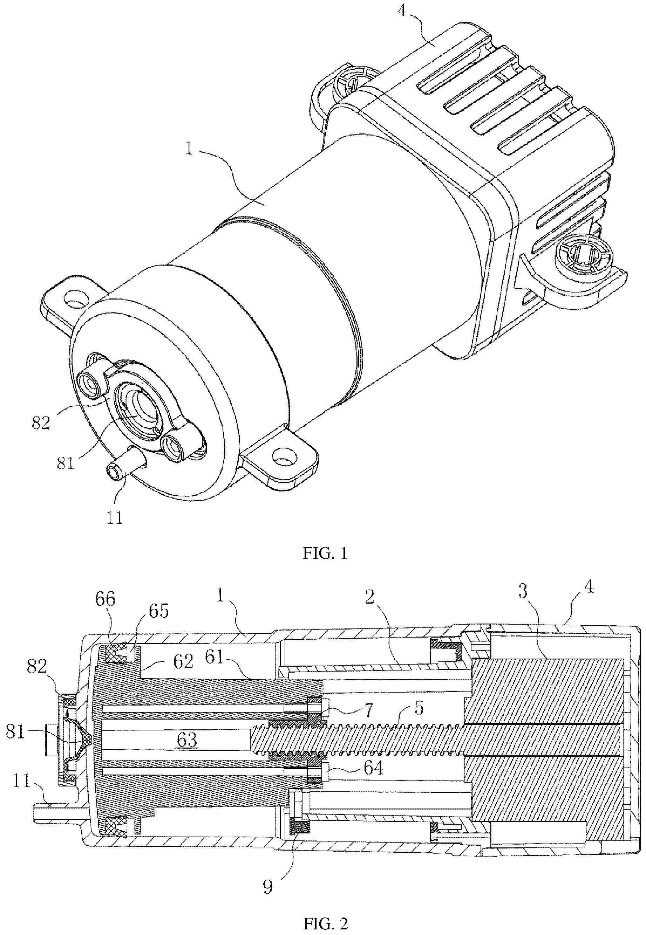

FIG. 1 is a structural schematic diagram of a breast pump driving apparatus;
FIG. 2 is a sectional structural schematic diagram of the breast pump driving apparatus;

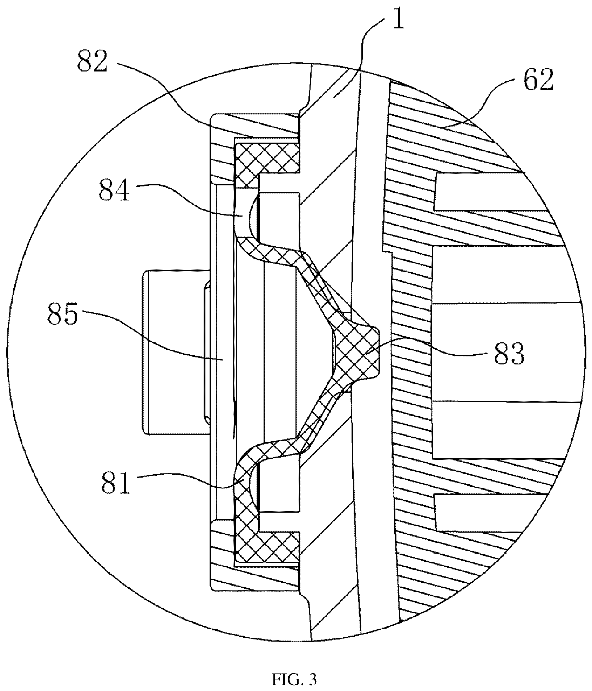

FIG. 3 is a partial structural schematic diagram of the breast pump driving apparatus shown in FIG. 2;

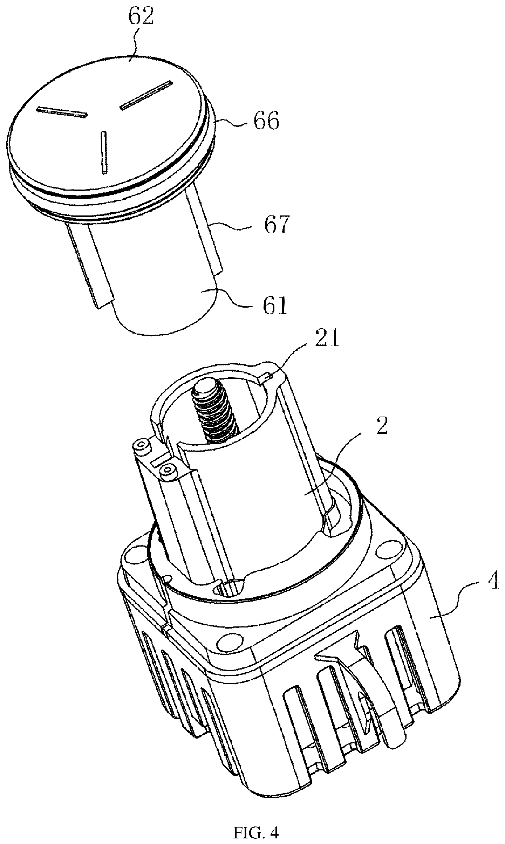

FIG. 4 is an exploded-view structural schematic diagram of a bracket and a piston;
FIGS. 5 and 6 are structural schematic diagrams of the bracket and a muffling airway provided on an the bracket;
FIG. 7 is a partial structural schematic diagram of the muffling airway shown in FIG. 6; and
FIG. 8 is a top-view structural schematic diagram of the muffling airway.

### SPECIFIC IMPLEMENTATIONS

The technical solutions in the embodiments of the present invention are described clearly and completely below with reference to the drawings. Apparently, the described embodiments are merely a part rather than all of the embodiments of the present invention. All other embodiments obtained by those of ordinary skill in the art based on the embodiments of the present invention without creative efforts should fall within the protection scope of the present invention.

It should be noted that all the directional indications (such as upper, lower, left, right, front, back, top, bottom, inner, outer, vertical, transverse, longitudinal, anticlockwise, clockwise, circumferential, radial, and axial) in the embodiments of the present invention are merely used to explain a relative position relationship or motion situations of the components in a specific gesture (as shown in the figures). If the specific gesture changes, the directivity indication also changes accordingly.

Moreover, the terms such as "first" and "second" described in the embodiments of the present invention are used only for the purpose of description and are not intended to indicate or imply relative importance, or implicitly indicate the number of the indicated technical features. Therefore, features defined by "first" and "second" may explicitly or implicitly include at least one of the features. Further, the technical solutions of various embodiments may be combined with each other, but it must be on the basis that a combination thereof can be implemented by those of ordinary skill in the art. In case of a contradiction with the combination of the technical solutions or a failure to implement the combination, it should be considered that the combination of the technical solutions does not exist, and is not within the protection scope of the present invention.

The present invention proposes a breast pump driving apparatus.

In an embodiment of the present invention, the breast pump driving apparatus includes housing 1 that is cylindrical and has an open end. The open end of the housing is provided with stepper motor 3 through bracket 2. The housing is provided with hood 4 for covering the stepper motor. An output shaft of the stepper motor is screw 5. A piston moving along an inner wall of the housing is threaded to the screw through screw nut 7. An end of the housing facing away from the stepper motor is provided with air tube joint 11. The air tube joint is provided with a throughh hole communicated with an inner cavity of the housing.

In the breast pump driving apparatus, the housing is hollow inside. The piston is hermetically connected to the inner wall of the housing through a sealing ring, and moves along the inner wall of the housing under the drive of the motor, thereby changing the volume of a chamber and achieving suction and exhaust. The motor is a stepper motor, which is connected to the piston through a screw-nut pair, allowing the piston to precisely control its stroke and change the volume of the chamber to form a specific suction. The air tube joint on the housing is connected to a milk bottle through an air tube, and a flange of the milk bottle fits tightly with a breast to form a seal. When the stepper motor drives the piston to move towards one end of the stepper motor, a negative pressure is generated inside the inner cavity of the housing, the air tube, and the milk bottle that are communicated with each other, thereby forming a suction to suck up breast milk. When the stepper motor drives the piston to move away from the stepper motor, a positive pressure is generated in the inner cavity of the housing. With the movement of the piston, air is discharged out of the housing through a one-way valve, restoring air pressure balance between the housing and the milk bottle to wait for a next milk suction action.

In the breast pump driving apparatus, the end of the housing 1 facing away from the stepper motor 3 is provided with an exhaust hole. The exhaust hole is provided with a one-way valve for allowing only air to be discharged outward. In addition, limit switch 9 is provided in the housing 1. The stepper motor 3 is started to drive the piston to move to a set position. When the limit switch detects that the piston is in place, a control system starts working. Thus, after the breast pump starts, a resetting operation is enabled to ensure an accurate and stable suction during subsequent operation, thereby improving working accuracy and user comfort. During startup, if the piston is located at one end of the stepper motor or in a middle of the housing, when the piston resets and moves away from the stepper motor, a positive pressure is generated in the inner cavity of the housing. Excess air is discharged through the one-way valve to ensure the operational safety of the breast pump. The one-way valve includes flexible diaphragm 81 and pressure plate 82 for fixing the diaphragm to the housing 1. The diaphragm is annular, and sealing part 83 is provided at a middle of the diaphragm and configured to seal the exhaust hole of the housing. The pressure plate is pressed against an annular edge of the diaphragm. The diaphragm is provided with diaphragm hole 84 located between the edge and the sealing part. The pressure plate is provided with pressure plate hole 85 for communicating two sides inside. When the piston moves towards the stepper motor, a negative pressure is generated in the inner cavity of the housing, thereby generating suction. The diaphragm is pressed against the exhaust hole of the housing through the sealing part under the action of the suction, thereby forming a seal on the exhaust hole. When the piston moves away from the stepper motor, a positive pressure is generated in the inner cavity of the housing, and there is a need for the air inside the housing to be discharged outward. The diaphragm deforms under the action of air thrust, and the sealing part is pushed away from the exhaust hole. Thus, the air inside the housing is discharged outward through the exhaust hole, the diaphragm hole, and the pressure plate hole, restoring air pressure balance in the housing and the milk bottle. This type of one-way valve has a simple structure but a very fast response speed to changes in air pressure, improving the working accuracy of the breast pump.

In the breast pump driving apparatus, the piston includes piston rod 61 and piston head 62. An end of the piston rod is provided with piston hole 63 for the screw 5 to extend into. The screw nut 7 is fixedly provided at the piston hole through fastening screw 64. The design ensures the sealing between the piston and the housing, and makes the connection structure between the parts compact to reduce the device volume. A side wall of the piston head 62 is provided with annular positioning groove 65 recessed inwardly. Sealing ring 66 is provided in the positioning groove. The sealing ring hermetically abuts againstthe inner wall of the housing 1 to form an interference fit, such that the sealing ring improves the sealing performance. In addition, the bracket 2 is sleeved on the piston rod 61. Positioning slot 21 and positioning rib 67 cooperating with each other are provided between an inner side of the bracket and an outer wall of the piston rod. the positioning slot and the positioning rib are parallel to an axial direction of the housing, thereby forming a limit and guide for the piston rod. The design improves the working stability and accuracy of the piston rod, and avoids erroneous rotation of the piston rod driven by the stepper motor.

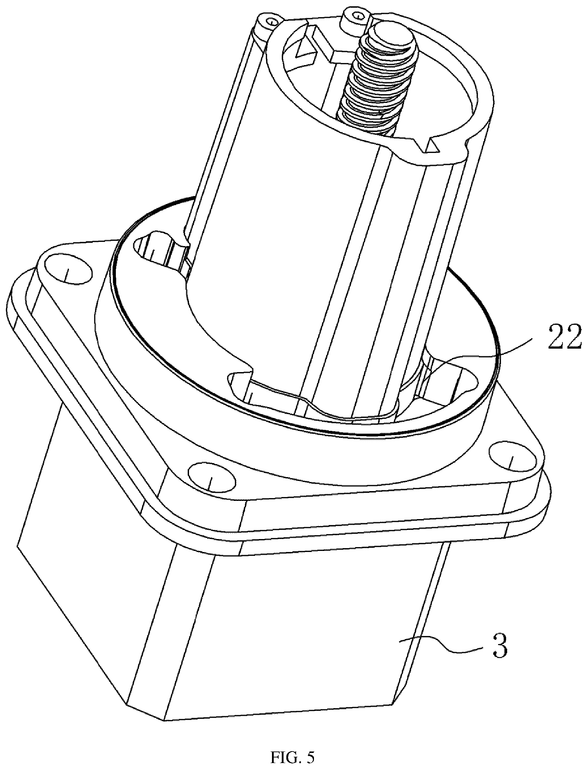

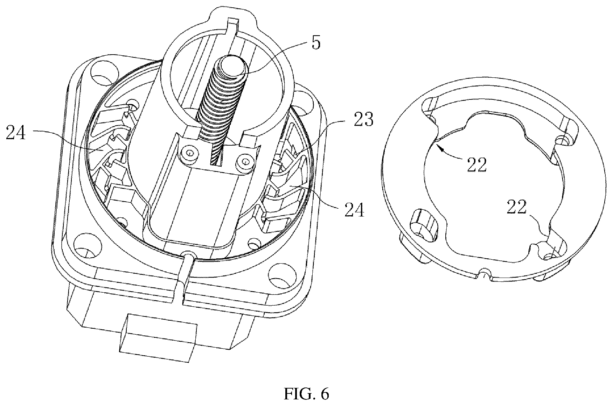

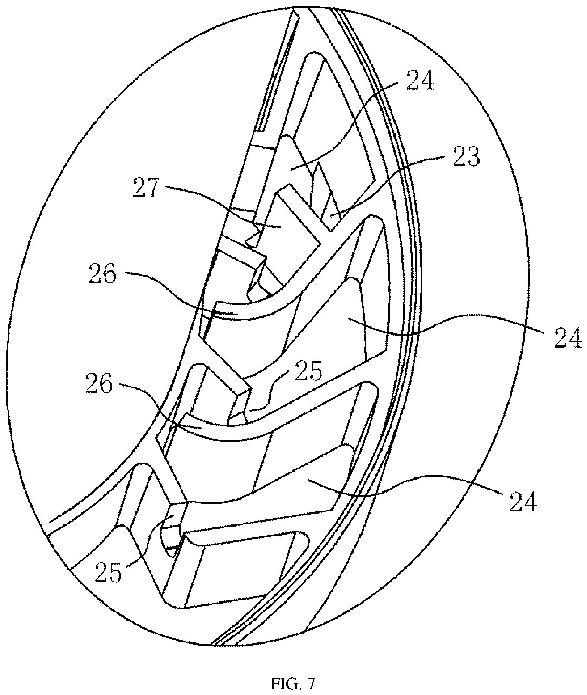

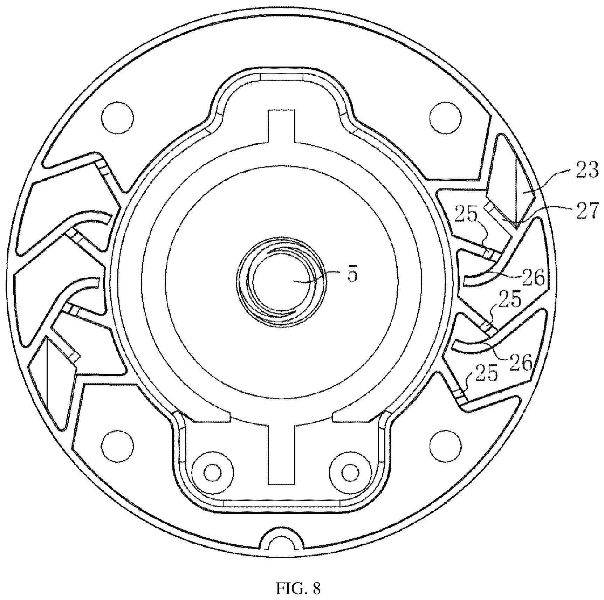

In the breast pump driving apparatus, an end of the bracket 2 is hermetically connected to the open end of the housing 1. A muffling airway is provided in the end of the bracket and configured to reduce a volume of noise. Sides of the end of the bracket facing and away from the piston are respectively provided with airway inlet 22 and airway outlet 23 that are communicated with the muffling airway. The main sources that generate noise in the breast pump driving apparatus are the screw-nut pair, the piston, and the airflow inside the housing. Considering this, the end of the bracket is sealed with the housing to encapsulate the main sources that generate noise inside chambers, effectively reducing the working noise of the breast pump. Meanwhile, through the muffling airway on the bracket, the sound wave and airflow undergo a noise reduction procedure through the muffling airway and are discharged out of the housing through the airway outlet, thereby further reducing the volume of the noise. The muffling airway includes a plurality of muffling chambers 24 that are sequentially connected, with a tail-end muffling chamber communicated with the airway outlet 23. Walls of all the muffling chambers facing the airway inlet 22 are respectively provided with muffling holes 25. All the muffling holes face respective outer sides of the muffling chambers (or towards an inner side of the muffling chamber), that is, towards a side wall of the muffling chamber facing away from the screw. In this way, the sound wave entering through the muffling hole can be directed towards an outer wall of the muffling chamber. On the one hand, the sound wave can pass through the entire muffling chamber before entering the next muffling chamber. On the other hand, the sound wave reflected by the outer wall of the muffling chamber will interfere with the incoming sound wave in the opposite direction, thereby weakening the sound wave to a certain extent. Therefore, the sound wave passing through all the muffling chambers and discharged from the airway outlet is greatly weakened, thereby reducing the noise. Specifically, as shown in FIGS. 5 to 8, the muffling chamber 24 includes "<"-shaped walls facing and away from the airway inlet 22. Upper and lower ends of the "<"-shaped wall are respectively connected to inner and outer walls of the muffling chamber. The muffling hole is located in an upper part of the "<"-shaped wall. A midpoint position of the "<"-shaped wall of the muffling chamber facing away from the airway inlet is provided with muffling plate 26 extending towards the inner wall of the muffling chamber. A gap is formed between the muffling plate and the inner wall of the muffling chamber, and a width of the gap is not greater than one tenth of a width of the muffling chamber. In this way, the muffling chamber can be further divided into two chambers, a large chamber and a small chamber. The large chamber has an outer width greater than an inner width thereof. The sound wave is directed from the muffling hole towards the outer side and then reflected towards the inner side, causing multiple reflections and mutual reflections, thereby weakening the sound wave multiple times. The weakened sound wave enters the small chamber through the gap at the muffling plate, and then is directed towards the next muffling chamber. Thus, a multi-stage muffling structure is formed, which greatly reduces the volume of the noise. In addition, the airway outlet 23 is located at a bottom of the tail-end muffling chamber 24 adjacent to an outer wall of the tail-end muffling chamber. The lower end of the "<"-shaped wall of the tail-end muffling chamber facing the airway inlet 22 is provided with partition 27 extending towards another wall, which can further reduce the volume of the noise. This muffling airway structure effectively reduces the working noise of the breast pump, thereby improving the user comfort.

The foregoing are merely preferred embodiments of the present invention, and the scope of the present invention is not limited thereto. Any equivalent structure change made using the content of the specification of the present invention and the drawings under the inventive concept of the present invention, or direct/indirect application thereof in other related technical fields, shall fall within the protection scope of the present invention.

## Claims

1. A breast pump driving apparatus, comprising a housing (1) that is cylindrical and has an open end, wherein a motoris provided at the open end of the housing through a bracket (2); the housing is provided with a hood (4) for covering the stepper motor; the motor is a stepper motor (3); an output shaft of the stepper motor is a screw (5); a piston moving along an inner wall of the housing is threaded to the screw through a screw nut (7); an end of the housing facing away from the stepper motor is provided with an air tube joint (11); and the air tube joint is provided with a throughh hole communicated with an inner cavity of the housing.

2. The breast pump driving apparatus according to claim 1, wherein the end of the housing (1) facing away from the stepper motor (3) is provided with an exhaust hole; the exhaust hole is provided with a one-way valve for allowing only air to be discharged outward; a limit switch (9) is provided in the housing (1); the stepper motor (3) is started to drive the piston to move to a set position; and when the limit switch detects that the piston is in place, a control system starts working.

3. The breast pump driving apparatus according to claim 2, wherein the one-way valve comprises a flexible diaphragm (81) and a pressure plate (82) for fixing the diaphragm to the housing (1); the diaphragm is annular, and a sealing part (83) is provided at a middle of the diaphragm and configured to seal the exhaust hole of the housing; the pressure plate is pressed against an annular edge of the diaphragm; the diaphragm is provided with a diaphragm hole (84) located between the edge and the sealing part; and the pressure plate is provided with a pressure plate hole (85) for communicating two sides inside.

4. The breast pump driving apparatus according to claim 1, 2 or 3, wherein the piston comprises a piston rod (61) and a piston head (62); an end of the piston rod is provided with a piston hole (63) for the screw (5) to extend into; and the screw nut (7) is fixedly provided at the piston hole.

5. The breast pump driving apparatus according to claim 4, wherein a side wall of the piston head (62) is provided with an annular positioning groove (65) recessed inwardly; a sealing ring (66) is provided in the positioning groove; and the sealing ring hermetically abuts againstthe inner wall of the housing (1).

6. The breast pump driving apparatus according to claim 4, wherein the bracket (2) is sleeved on the piston rod (61); a positioning slot (21) and a positioning rib (67) cooperating with each other are provided between an inner side of the bracket and an outer wall of the piston rod; and the positioning slot and the positioning rib are parallel to an axial direction of the housing.

7. The breast pump driving apparatus according to claim 1, 2 or 3, wherein an end of the bracket (2) is hermetically connected to the open end of the housing (1); a muffling airway is provided in the end of the bracket and configured to reduce a volume of noise; and sides of the end of the bracket facing and away from the piston are respectively provided with an airway inlet (22) and an airway outlet (23) that are communicated with the muffling airway.

8. The breast pump driving apparatus according to claim 7, wherein the muffling airway comprises a plurality of muffling chambers (24) that are sequentially connected, with the last muffling chamber communicated with the airway outlet (23); walls of all the muffling chambers facing the airway inlet (22) are respectively provided with muffling holes (25); and all the muffling holes face respective outer sides of the muffling chambers.

9. The breast pump driving apparatus according to claim 8, wherein the muffling chamber (24) comprises "<"-shaped walls facing and away from the airway inlet (22); upper and lower ends of the "<"-shaped wall are respectively connected to inner and outer walls of the muffling chamber; the muffling hole is located in an upper part of the "<"-shaped wall; a midpoint position of the "<"-shaped wall of the muffling chamber facing away from the airway inlet is provided with a muffling plate (26) extending towards the inner wall of the muffling chamber; a gap is formed between the muffling plate and the inner wall of the muffling chamber; and a width of the gap is not greater than one tenth of a width of the muffling chamber.

10. The breast pump driving apparatus according to claim 9, wherein the airway outlet (23) is located at a bottom of the tail-end muffling chamber (24) adjacent to an outer wall of the tail-end muffling chamber; the lower end of the "<"-shaped wall of the tail-end muffling chamber facing the airway inlet 22 is provided with a partition (27) extending towards another wall.
